# EUROPÄISCHE PATENTSCHRIFT

(11) **EP 1 076 648 B1**
(45) Veröffentlichungstag und Bekanntmachung des Hinweises auf die Patenterteilung: **12.07.2006**
(21) Anmeldenummer: 99920777.2
(22) Anmeldetag: 24.04.1999
(51) Int. Cl.: C07D 213/22, C07D 213/80, C07D 213/79, C07D 213/30, C07D 215/04, C07B 37/02

(54) **VERFAHREN ZUR KATALYTISCHEN HERSTELLUNG VON SUBSTITUIERTEN BIPYRIDYLDERIVATEN**
METHOD FOR THE CATALYTIC PRODUCTION OF SUBSTITUTED DIPYRIDYL DERIVATIVES
PROCEDE POUR LA PRODUCTION CATALYTIQUE DE DERIVES DE BIPYRIDYLE SUBSTITUES

(30) Priorität: 29.04.1998 DE 19819010
(43) Veröffentlichungstag der Anmeldung: 21.02.2001
(73) Patentinhaber: Merck Patent GmbH, 64293 Darmstadt (DE)
(72) Erfinder: GEISSLER, Holger, D-55116 Mainz (DE); GROSS, Peter, D-65451 Kelsterbach (DE)
(86) Internationale Anmeldenummer: PCT/EP1999/002788
(87) Internationale Veröffentlichungsnummer: WO 1999/055675

(56) Entgegenhaltungen:
- EP-A- 0 206 543
- DE-A- 2 230 562
- P. BAMFIELD ET AL.: "A new synthesis of biaryls from aryl halides using aqueous alkaline sdium formate with palladium on charcoal and surfactant as catalyst" SYNTHESIS., Nr. 7, 1978, Seiten 537-538, XP002107555 STUTTGART DE in der Anmeldung erwähnt

## Beschreibung

Die vorliegende Erfindung betrifft ein verbessertes Verfahren zur katalytischen Herstellung von substituierten Bipyridylderivaten durch Umsetzung von substituierten Halogenpyridinderivaten mit einer Base und einem Reduktionsmittel in Gegenwart eines Palladium-Katalysators..

Ein derartiges Verfahren, welches für Bipyridyle der Reaktionsgleichung 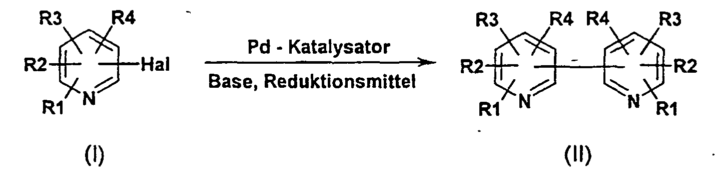 folgt, wurde bei Umsetzung mit Chlorpyridinen häufig in geringen Ausbeuten und mit den wesentlich teureren und damit industriell weniger interessanten Brompyridinen und lodpyridinen in höheren Ausbeuten in den nachfolgend aufgeführten Publikationen beschrieben. Die bisher bekannten Verfahren weisen ökonomische und/oder ökologische Nachteile auf.

Bipyridyle, die in der Literatur häufig auch als Bipyridine bezeichnet werden, einschließlich ihrer Derivate sind z.B. als industrielle und medizinische Intermediate, als analytische Reagenzien oder als Liganden für die Darstellung von Metallkomplexen mit katalytischer Aktivität wichtig. Die Verwendung für Bipyridyl-Metallkomplexe, besonders von Rutheniumkomplexen, ist aufgrund der Anwendung als Photosensibilisatoren zur Sensibilisierung von Halbleiteroberflächen, als Photokatalysator für Solarzellen, insbesondere für Photovoltaische Zellen bzw. Photoelektrochemische Zellen wie z.B. in der WO 91/16719 oder EP-A-0 333 641 beschrieben und für die photoinduzierte Elektrolyse von besonderer Bedeutung.

Die Synthese von substituierten Bipyridylen aus Halogenpyridinen gelingt nach dem Stand der Technik in der Regel nur mit guten Ausbeuten, wenn stöchiometrische Mengen einer Metallverbindung eingesetzt werden. Bei der Anwendung von katalytischen Mengen dieser Metallverbindungen ist die Ausbeute dagegen meist gering, und die Brompyridine bzw. die lodpyridine werden als Startmaterialien verwendet.

Tiecco und Testaferri beschreiben in Synthesis 1984, 736ff, die Synthese von Bipyridylderivaten unter Einsatz von stöchiometrischen Mengen an elementarem Zink und Nickelchlorid sowie vier Äquivalenten Triphenylphosphin in Dimethylformamid als Lösemittel bei 50 °C. Mit diesem Verfahren kann z.B. 3-Bromo-2-methoxy-pyridin in einer Ausbeute von 75 % mit einem Äquivalent Zink-(II)-chlorid sowie einem Äquivalent Nickel-(II)-chlorid als Nebenprodukt umgesetzt werden. In zwei Vergleichsversuchen, in denen Palladium auf Aktivkohle als Katalysator bzw. Kupfer zum Einsatz kommt, kann das Produkt 3,3'-Dimethoxy-2,2'-bipyridyl nur in einer Ausbeute von 18 % bzw. 23 % erhalten werden; die Versuchsbedingungen sind in Synthesis 1978, 537ff, beschrieben. Beim Einsatz von katalytischen Mengen von Nickel-(II)-chlorid wie von Zembayashi et al. in Tetrahedron Lett. 1977, 4089ff, veröffentficht, wird hauptsächlich das dehalogenierte Startmaterial und nur in geringer Ausbeute das gewünschte Produkt erhalten.

Mit dem von Tiecco und Testaferri beschriebenen Verfahren können auch Chlorpyridine umgesetzt werden, wobei jedoch gleichzeitig hohe Mengen an Schwermetallsalzen anfallen, so daß dieses Verfahren weder ökologisch, noch ökonomisch attraktiv ist. Zum Beispiel wird 2-Chlor-5-methoxy-pyridin in einer Ausbeute von 88 % zu 5,5'-Dimethoxy-3,3'-bipyridyl mit einem Äquivalent Zink-(II)-chlorid sowie einem Äquivalent Nickel-(11)-chlorid als Nebenprodukt umgesetzt.

Langhals offenbart in DE-A-39 21 025, daß die von Tiecco und Testaferri beschriebene Reaktion auch in Gegenwart freier phenolischer Hydroxy-Gruppen erfolgen kann. Langhals offenbart gleichzeitig, daß das bei der Reaktion als Nebenprodukt anfallende Zinkhydroxid in den Beispielen zur Synthese des 3,3'-Dihydroxy-2,2'-bipyridyls und des 3,3'-Dihydroxy-2,2'-bichinolins nur schwer abtrennbar ist.

Newcame gibt in J. Inorg. Nucl. Chem. 1981, Vol. 43, 1529ff, die Umsetzung von methylsubstituiertem Brompicolin zu dem korrespondierenden Bipyridyl unter Katalyse von Palladium auf Aktivkohle und einem Phasen-Transfer-Reagenz in Gegenwart von Natriumformiat als Reduktionsmittel im Zweiphasensystem bekannt. Mit diesem Verfahren kann z.B. 2-Brom-6-methylpyridin (= 2-Brom-6-picolin) in einer Ausbeute von 59 % zu 3,3'-Dimethyl-2,2'-bipyridyl umgesetzt werden. Der Nachteil dieser Methode ist der zwingend notwendige Einsatz eines Phasen-Transfer-Reagenzes als Cokatalysator neben Palladium. Ebenso kommen für diese Methode üblicherweise nur Brompyridine, die in der Regel mit Alkylgruppen substituiert sind, als Startmaterialien zum Einsatz.

Die klassische Ullmann-Reaktion, wie sie von Fanta in Synthesis 1974, 9ff, beschrieben wird, verlangt äußerst drastische Reaktionsbedingungen und den Einsatz stöchiometrischer Mengen an Kupfer als Reduktionsmittel. Sie ergibt in den meisten Fällen nur geringe Ausbeuten der gewünschten Bipyridyle bzw. von deren Derivaten. Die Ullmann-Reaktion ist nach Fanta für die Synthese von Bipyridylen, die mit Amino-, Hydroxy- und/oder freien Carboxyl-Gruppen substituiert sind, nicht anwendbar.

Die Synthese von Carboxy-substituierten Bipyridylen der allgemeinen Formel (IIa) 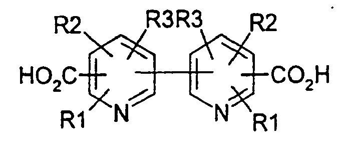 stellt gegenwärtig ein besonderes Problem dar. In der Regel wird diese Substanzklasse durch Oxidation der leichter zugänglichen Dimethyl-substituierten Bipyridyfe mit Kaliumpermanganat erhalten, wie dies z.B. von Bruce in Liquid Crystals, 1996, Vol. 20, Seite 183-193 beschrieben wird.
Shaw offenbart in DE-A-24 50 100, daß Bipyridyle und mit Alkyl-, Amino-, Cyano- und/oder Carboxyl-Gruppe substituierte Bipyridyle in Ausbeuten von nur 50 % auch durch ein elektrochemisches Verfahren aus den entsprechenden Pydridinen zugänglich gemacht werden können. Die große Zahl der Nebenprodukte steht einer technischen und ökologisch sowie ökonomisch attraktiven Realisierung im Wege. Shaw offenbart gleichzeitig, daß bei den anderen bekannten Verfahren zur Herstellung von Bipyridylen häufig als Zwischenprodukte Metallderivate des Pyridins, vor allem Natriumderivate, hergestellt werden müssen, so daß die Herstellung von Bipyridylen nach solchen Verfahren gefährlich und vergleichsweise teuer ist.

Badger und Sasse beschreiben in J. Chem. Soc. 1956, 616ff, die Umsetzung von 50 g Nicotinsäure mit aktiviertem Nickel, das aus 125 g Nickellegierung hergestellt wurde, zu nur 1 g des gekuppelten Produktes. Die geringe Ausbeute dieses Verfahrens machen das Verfahren für die Synthese dieser Substanzklasse nicht interessant.

US-A-3,767,652 lehrt, daß 2,2'-Bis(3-pyridinole) durch oxidative Kupplung von den entsprechenden Pyridinolen mit stöchiometrischen Mengen Bleidioxid als Oxidationmittel in geringen Ausbeuten bis zu 35 % dargestellt werden können. Die Anwendung stöchiometrischer Mengen an Bleisalzen macht dieses Verfahren ökologisch bedenktich.

Die Synthese von Bipyridylen ohne Substituenten gelingt nach DE-A-22 30 562 mit Ausbeuten bis zu 65 % durch Umsetzung von Chlor- oder Brompyridin in Methanol in Gegenwart von Wasser, einem reduzierend wirkenden Additiv wie z.B. Hydroxylamin oder Hydrazin, einer Base wie z.B. Kaliumhydroxid und geträgertem Palladium. Die Synthese von Bipyridylen mit Alkylgruppen als Substituenten, die in der Offenlegungsschrift als inerte Substituenten bezeichnet werden, ist nach dem beschriebenen Verfahren jedoch nur in Ausbeuten von bis zu 26 % gelungen. Der große Nachteil des Verfahrens liegt in der hohen Umsetzung zu dehalogenierten Pyridinen, die bis zu 68 % betragen kann. Die Dehalogenierung scheint durch große Mengen an Wasser im Reaktionsmedium begünstigt zu werden.

EP-A-O 206 543 offenbart die Synthese von Bipyridyl durch Umsetzung von einem Halogenpyridin in Gegenwart von 0,2 bis 3 MPa Kohlenmonoxid, einem alkalischen Medium und einem geträgerten Palladiumkatalysator. Die Verwendung von Kohlenmonoxid ist jedoch aufgrund der hohen sicherheitstechnischen Anforderungen für den Umgang mit diesem hochtoxischen Gas für eine technische Realisierung unökonomisch.

Da die bisher beschriebenen Verfahren zur Darstellung von substituierten Bipyridylen nur mit hoher ökologischer Belastung in guten Ausbeuten durchführbar sind, bestand ein hoher Bedarf an einem Verfahren, das substituierte Bipyridyle und besonders Carboxy-substituierte Bipyridyle in hoher Ausbeute und Reinheit in technisch einfach durchführbarer und ökologischer Weise zugänglich macht. Es bestand die Aufgabe, ein derartiges Verfahren zu entwickeln.

Diese Aufgabe wird gelöst durch ein Verfahren zur Herstellung von Bipyridyien durch Umsetzung von mindestens einem Pyridin der allgemeinen Formel (III) 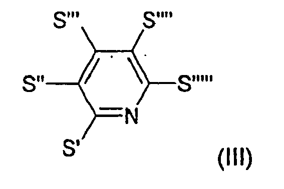 gemäß Patentanspruch 1.

Besonders bevorzugt bedeuten S', S", S"', S"" und S""' unabhängig voneinander gleich oder verschieden Wasserstoff, Methyl, Ethyl, Isopropyl, Methoxy, Acetoxy, Trifluoracetoxy, Trifluormethyl, O-Phenyl, Phenyl, Fluor, OH, Nitro, Nitroso, CN, COOH, CHO, PO₃H₂, SO;H, NH₂, NH-Alkyl-(C₁-C₈), N-Alkyl₂-(C₁-C₈), NHCO-Alkyl-(C₁-C₄), N-Alkyl-(C₁-C₄)-CO-Alkyl-(C₁-C₄), CONH₂, CO-Alkyl-(C₁-C₈), NHCOH, CO-Phenyl, CHCHCO₂H, PO-Phenyl₂, PO-Alkyl₂-(C₁-C₄), (COO⁻)ₙ(Kation)ⁿ⁺. (PO₃²⁻)ₙ(Kation)2ⁿ⁺, (SO₃⁻)ₙ(Kation)ⁿ⁺ oder/und (O⁻)ₙ(Kation)ⁿ⁺,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet
und wobei mindestens einer der Substituenten von S', S", S"', S"" und S""' ein nicht inerter Substituent wie OH, COOH, PO₃H₂ oder SO₃H, (COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺ ist,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet,
und wobei mindestens einer der Substituenten von S', S", S"', S"" und S""' Chlor, Brom oder Jod ist,
wobei gegebenenfalls S', S", S"', S"" oder/und S""' untereinander zusammen einen oder mehrere aliphatische oder/und aromatische Ringe bilden
oder/und worin gegebenenfalls S', S", S"', S"" oder/und S""' eine Brücke zu mindestens einem weiteren Pyridin der allgemeinen Formel (III) bildet
oder/und worin gegebenenfalls die Reste S', S", S"', S"" oder/und S""' die oben angegebene Bedeutung besitzen und mit mindestens einem Rest, der die oben angegebene Bedeutung für S', S", S"', S"" oder/und S""' besitzt, substituiert ist.

Das Verfahren eignet sich gut zur Herstellung von symmetrischen 2,2'-Bipyridylen der allgemeinen Formel (IV), symmetrischen 3,3'-Bipyridylen der allgemeinen Formel (V) oder symmetrischen 4,4'-Bipyridylen der allgemeinen Formel (VI), 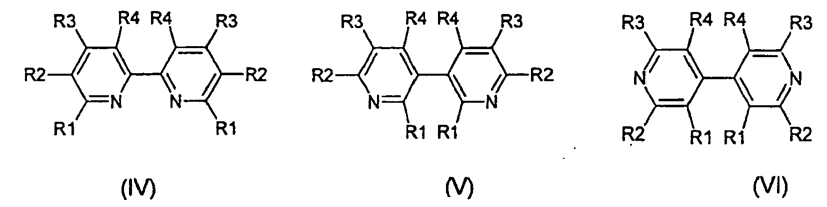 gemäß Patentanspruch 3.

Als geschütztes Amin sind Amine mit Schutzgruppen zu verstehen, wie sie z.B. in Greene, Theodora W.; Wuts, Peter G. M. *Protective Groups in Organic Synthesis,* 2^{nd} Ed. 1991, Publisher: John Wiley and Sons, Inc., New York, N.Y., beschrieben sind. Auf diese Publikation wird ausdrücklich Bezug genommen.

Bei der Herstellung dieser symmetrischen Bipyridyle entsteht aus dem Halogenpyridin der allgemeinen Formel (VII) das symmetrische 2,2'-Bipyridylen der allgemeinen Formel (IV), aus dem Halogenpyridin der allgemeinen Formel (VIII) das symmetrische 3,3'-Bipyridylen der allgemeinen Formel (V) und aus dem Halogenpyridin der allgemeinen Formel (IX) das symmetrische 4,4'-Bipyridylen der allgemeinen Formel (V1).

Bevorzugt bedeuten:
R1 bis R4 unabhängig voneinander Wasserstoff, C₁-C₈-Alkyl, Alkoxy-(C₁-C₈), Acyloxy-(C₁-C₈), Aryloxy, Aryl, Heteroaryl, Fluor, Hydroxy, Nitro, Nitroso, CN, COOH, CHO, PO₃H₂, SO₃H, SO₂R, SOR, NH₂, NH-Alkyl-(C₁-C₈), N-Alkyl₂-(C₁-C₈), geschütztes Amin, CF₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH; NHCOO-Alkyl-(C₁-C₄), CO-Aryl, COO-Aryl, CHCH-CO₂-Alkyl-(C₁-C₈), CHCHCO₂H, PO-Phenyl₂, PO-Alkyl₂-(C₁-C₄), (COO⁻)ₙ(Kation)ⁿ⁺,
(PO₃²⁻)ₙ(Kation)₂ⁿ⁺, (SO₃⁻)ₙ(Kation)ⁿ⁺ oder/und (O⁻)ₙ(Kation)ⁿ⁺,
wobei Kation Na, Li, K, Ca, Mg, NR₃H, NR₄, NH₄, PR₃H, PR₄ oder/und PH₄ und Aryl, Aryloxy und Heteroaryl mit bis zu 18C-Atomen bedeutet, und
wobei R Aryl mit bis zu 18C-Atomen oder/und, (C₁-C₁₈)-Alkyl unabhängig voneinander gleich oder verschieden bedeutet und wobei mindestens einer der Substituenten von R1 bis R4 ein nicht inerter Substituent wie Acyloxy-(C₁-C₈), Hydroxy, COOH, PO₃H₂, SO₃H, SO₂R, NH₂, NH-Alkyl-(C₁-C₈), N-Alkyl₂-(C₁-C₈), geschütztes nicht inertes Amin, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), COO-Aryl mit bis zu 18 C-Atomen, CHCH-CO₂₋Alkyl-(C₁-C₈), CHCHCO₂H, (COO⁻)ₙ(Kation)ⁿ⁺, (PO₃²⁻)n(Kation)₂ⁿ⁺, (SO₃⁻)ₙ(Kation)ⁿ⁺ oder/und
(O⁻)ₙ(Kation)ⁿ⁺ ist,
wobei Kation Na, Li, K, Ca, Mg, NR₃H, NR₄, NH₄, PR₃H, PR₄ oder/und PH₄ ist und
wobei R Aryl mit bis zu 18 C-Atomen oder/und (C₁-C₁₈)-Alkyl unabhängig voneinander gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

Als nicht inerte Substituenten werden solche angesehen, die mit mindestens einer Verbindung im Reaktionsmedium eine Reaktion eingeht, so daß stabile Addukte oder aber der Substituent als solcher verändert werden kann. So ist z.B. eine COOH-Gruppe als nicht inert anzusehen, da sie spontan im Reaktionsmedium eine Säure-Base-Reaktion eingeht oder auch eine COONa-Gruppe, da sie als basische Verbindung eine Reaktion mit der entstehenden Säure, die in der Regel durch Basenzusätze abgefangen wird, eingehen kann.

Besonders bevorzugt bedeuten:
R1 bis R4 unabhängig voneinander Wasserstoff, Methyl, Ethyl, Tert.-Butyl, Isopropyl, Methoxy, Acetoxy, Trifluoracetoxy, Trifluormethyl, O-Phenyl, Phenyl, Fluor, OH, Nitroso, NO₂, CN, COOH, CHO, PO₃H₂, SO₃H, NH₂, NH-Alkyl (C₁-C₈), N-Alkyl₂-(C₁-C₈), NHCO-Alkyl-(C₁-C₄), N-Alkyl-(C₁-C₄)-CO-Alkyl-(C₁-C₄), COO-Alkyl-(C₁₋C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), CHCHCO₂H, PO-Phenyl₂, PO-Alkyl₂-(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und
(O⁻)ₙ(Kation)ⁿ⁺,
wobei Kation Na, Li, K, NR₃H, NR₄, PR₃H oder/und PR₄ und
wobei R Aryl mit bis zu 18 C-Atomen oder/und (C₁-C₁₈)-Alkyl unabhängig voneinander gleich oder verschieden bedeutet
und wobei mindestens einer der Substituenten von R1 bis R4 ein nicht inerter Substituent wie Acetoxy, Trifluoracetoxy, OH, COOH, PO₃H₂, SO₃H, NH₂, NH-Alkyl-(C₁-C₈), N-Alkyl₂-(C₁-C₈), NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), COO-Phenyl, CHCH-CO₂-Alkyl-(C₁-C₈), CHCHCO₂H, (COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺ ist,
wobei Kation Na, Li, K, NR₃H, NR_{d}, PR₃H oder/und PR₄ und
wobei R Aryl mit bis zu 18 C-Atomen oder/und (C₁-C₁₈)-Alkyl unabhängig voneinander gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

Ganz besonders bevorzugt bedeuten:
R1 bis R4 unabhängig voneinander Wasserstoff, Methyl, Ethyl, Isopropyl, Methoxy, Acetoxy, Trifluoracetoxy, Trifluormethyl, Trichlormethyl, O-Phenyl, Phenyl, Fluor, OH, Nitroso, NO₂, CN, COOH, CHO, PO₃H₂, SO₃H, NH₂, NH-Alkyl-(C₁-C₈), N-Alkyl₂₋(C₁-C₈), NHCO-Alkyl-(C₁-C₄), N-Alkyl-(C₁-C₄)-CO-Alkyl-(C₁-C₄), CONH₂, CO-Alkyl-(C₁₋C₈), NHCOH, CO-Phenyl, CHCHCO₂H, PO-Phenyl₂, PO-Alkyl₂-(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet
und wobei mindestens einer der Substituenten von R1 bis R4 ein nicht inerter Substituent wie OH, COOH, PO₃H₂ oder SO₃H, (COO⁻)ₙ(Kation)ⁿ⁺,
(PO₃²⁻)ₙ(Kation)₂ⁿ⁺, (SO₃⁻)ₙ(Kation)ⁿ⁺ oder/und (O⁻)ₙ(Kation)ⁿ⁺ ist,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

Es können ebenfalls die Salze der Halogenpyridine für die Umsetzung verwendet werden. So kann insbesondere ein Alkalisalz, z.B. das Natriumsalz, einer Halogenpyridincarbonsäure oder ein Hydrochlorid eines Halogenpyridins verwendet werden.

Die Reste R', R", R"' oder/und R"" können gegebenenfalls substituiert sein. Geeignete Substituenten sind die Reste R1 bis R4 selbst, insbesondere die nicht inerten Substituenten wie OH, COOH, PO₃H₂ oder/und SO₃H.

Beim Einsatz von zwei oder mehr verschiedenen Monohalogenpyridinen werden neben den symmetrischen Bipyridylen auch unsymmetrisch gekoppelte Bipyridyle erzeugt.

Überraschenderweise und entgegen den nach dem Stand der Technik zu erwartenden Ergebnissen wurde gefunden, daß sich Halogenpyridine mit nicht inerten Substituenten besonders gut in Wasser in Gegenwart eines Alkohols wie z.B. Methanol, einer Base wie z.B. Natriumhydroxid, eines Palladium-Katalysators und gegebenenfalls mindestens eines weiteren mit Wasser mischbaren oder nicht mischbaren Lösemittels, jedoch auch ohne weitere Additive wie z.B. reduzierenden Verbindungen wie Hydrazin oder Hydroxylamin, bei Temperaturen von 0 - 200 °C umsetzen lassen.

Im Fall der besonders bevorzugten Halogenpyridyle begünstigt ein hoher Wasseranteil im Reaktionsmedium (> 35 %) die Bildung der Bipyridyle, was nach dem Stand der Technik nicht zu erwarten war. Vorzugsweise wird ein Wasseranteil im Reaktionsmedium von mindestens 40 %, insbesondere von mindestens 50 % für die Bildung der Bipyridyle verwendet.

Als Palladium-Katalysator hat sich der Einsatz von trägerlosen und bevorzugt von geträgertem Palladium bewährt. Als Träger eignen sich die üblicherweise für Palladium eingesetzten Träger, insbesondere Aktivkohle, Metalloxide oder/und Metallsalze wie z.B. Sulfate oder/und Carbonate der Metalle der 2. bis 3. Hauptgruppe oder/und der 1. bis 3. Nebengruppe wie z.B. Aluminiumoxid, Bariumsulfat, Calciumcarbonat oder/und Siliciumdioxid und besonders bevorzugt Aktivkohle. Die Träger können in dem erfindungsgemäßen Verfahren auch in gemischter Form eingesetzt werden. Als Palladium-Katalysator eignet sich sowohl ein metallisches Palladium, als auch eine Palladium-Verbindung, gegebenenfalls in Kombination miteinander.

Überraschenderweise wurde gefunden, daß die Beladung des Palladiums auf dem Träger bei gleicher Gesamtmenge des eingesetzten Palladiums von wesentlicher Bedeutung für die Reaktionsgeschwindigkeit ist. So zeigt sich, daß 30 Gew.-% Palladium auf Aktivkohle um ein Vielfaches schneller reagiert als die gleiche Menge Palladium als 5 Gew.-% Palladium auf Aktivkohle. Insbesondere beträgt die Beladung des Palladiums auf dem Träger mindestens 5 Gew.%, vorzugsweise mindestens 10 Gew.-%, besonders bevorzugt mindestens 20 Gew.-%.
Das Verfahren der Erfindung ist vorzugsweise dadurch ausgezeichnet, daß man den Palladium-Katalysator, jeweils berechnet auf Palladiummetall, in einer Menge von 0,0001 bis 10 mol%, bevorzugt in einer Menge von 0,1 bis 5 mol%, in Bezug auf Halogenpyridin einsetzt. Wird mehr Palladium eingesetzt, so erhöht sich die Reaktionsgeschwindigkeit entsprechend, und man erhält eine höhere Raum-ZeitAusbeute. Insbesondere in einem Verfahren, bei dem der Palladium-Katalysator durch einfache Abtrennung wieder zurückgewonnen und wieder eingesetzt wird, ist die Verwendung großer Palladiummengen daher von Vorteil.

Der Alkohol dient primär als Reduktionsmittel in der Reaktion und kann zu den Folgeprodukten Aldehyd bzw. Keton, Carbonsäure und/oder Kohlendioxid bzw. Kohlensäure weiteroxidiert werden. Die in der Reaktion auftretenden Aldehyde bzw. Ketone können im alkalischen Reaktionsmedium im Sinne einer Aldolreaktion weiterreagieren. Als Alkohol werden daher vorzugsweise Alkohole verwendet, die nach der Oxidation zum Aldehyd bzw. Keton keine bzw. nur schwer eine Aldolreaktion eingehen können. Methanol oder/und Ethylenglykol haben sich für das erfindungsgemäße Verfahren besonders bewährt.

Als Base können beispielsweise Salze von schwachen und starken Säuren zum Einsatz kommen. Insbesondere können Alkali- oder/und Erdalkalisalze verwendet werden, vorzugsweise Alkalihydroxide, Erdalkalihydroxide, Alkalicarbonate, Erdalkalicarbonate, Alkalihydrogencarbonate, Erdalkalihydrogencarbonate, Alkaliacetate oder/und Erdalkaliacetate, besonders bevorzugt Natriumhydroxid oder/und Kaliumhydroxid.

Die Reaktion wird bei einer Temperatur von 0 bis 200 °C, insbesondere 10 bis 180 °C, vorzugsweise 20 bis 150 °C und besonders bevorzugt 50 bis 120 °C, durchgeführt. Die erfindungsgemäße Reaktion wird bevorzugt bei 0,1 bis 2 MPa, insbesondere bei 0,1 bis 0,5 MPa durchgeführt. Der erhöhte Druck dient im wesentlichen zur Erhöhung des Siedepunktes des verwendeten Lösemittels, um die optimale Reaktionstemperatur einstellen zu können.
Vorzugsweise enthält das Reaktionsprodukt 2,2'-Bipyridyl-4,4'-dicarbonsäure.

Beim Einsatz von Dihalogenpyridinen wie z.B. der 2,6-Dichlorisonicotinsäure entstehen neben den Bipyridylen auch Oligo- bzw. Polypyridyle, die in der Regel als Feststoff aus der Lösung ausfallen. Bei der Reaktion entstehen üblicherweise als Nebenprodukte auch die dehalogenierten Produkte. Die Kettenlänge der Oligo- und

Polypyridyle ist durch das Verhältnis der Reaktionsgeschwindigkeiten der reduktiven Dimerisierung zur Dehalogenierung bestimmt. Die Übertragung des erfindungsgemäßen Verfahrens auf Dihalogenpyridyle, welches für das Beispiel 2,6-Dichlorisonicotinsäure der Reaktionsgleichung 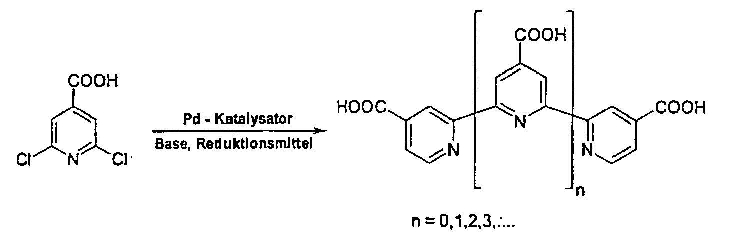 folgt, ist somit zur Synthese von Gemischen aus Bi-, Oligo- und Polypyridylen einsetzbar.

Vorzugsweise wird das Reaktionsprodukt des erfindungsgemäßen Verfahrens mit mindestens einem Übergangsmetall der 7. oder/und 8. Nebengruppe, vorzugsweise Mangan oder/und einem Edelmetall, insbesondere Ruthenium, oder/und deren Verbindungen zu mindestens einem Bipyridyl-Metallkomplex umgesetzt. Diese Übergangsmetallkomplexe eignen sich hervorragend als Sensibilisierungsfarbstoffe für farbstoffsensibilisierte Solarzellen. Insbesondere Ruthenium-Komplexe haben sich aufgrund ihrer Lichtabsorptions- und Elektroneninjektionseigenschaften in Halbleitern wie TiO₂ als besonders geeignet erwiesen. Als besonders geeignete Beispiele seien die Verbindungen cis-X₂Bis(2,2'-bipyridyl-4,4'-dicarboxylat)-Ruthenium(II) mit X = Cl-, Br-, l-, CN- oder SCN- genannt.
Solche Verbindungen lassen sich beispielsweise aus der Umsetzung wasser- oder methanollöslicher Bipyridylverbindungen oder ihrer Salze mit Metallsalzen gewinnen.

Ein derartiges Reaktionsprodukt kann daher hervorragend in photovoltaischen oder photoelektrochemischen Zellen, insbesondere in Solarzellen, oder für die photoinduzierte Elektrolyse eingesetzt werden.

Die folgenden Beispiele sollen das Verfahren erläutern.

### Beispiele

### Beispiel 1:

25 g 6-Chlornicotinsäure und 14 g Natriumhydroxid werden in einem Gemisch aus 100 ml Wasser und 80 ml Ethylenglycol gelöst. Nach der Zugabe von 0,56 g Palladium (30 Gew.% auf Aktivkohle; entspricht einer Zugabe von 168 mg reinen Palladiums) als Katalysator wird 5 h bei 80-85 °C bei 0,1 MPa gerührt. Anschließend wird der Katalysator abfiltriert. Nach dem Ansäuern auf pH 1 mit Salzsäure fällt das Produkt, 2,2'-Bipyridyl-5;5'-dicarbonsäure, als weißer Feststoff aus. Man erhält 15,7 g (81 % Ausbeute). Das Rohprodukt hat nach ¹H NMR eine Reinheit von ca. 95 %. CAS Registry Number: 1802-30-8.
¹H NMR (400 Mhz, DMSO):d = 13.51 (s breit, 2H); 9.20 (dd, J = 2.1, 0.8 Hz, 2 H) ; 8.57 (dd, J = 8.2, 0.8 Hz, 2 H) ; 8.45 (dd, J = 8.2, 2.1 Hz, 2 H).
¹³C NMR (100 Mhz, DMSO): d = 165.92, 157.29, 150.25 (CH), 138.38 (CH), 127.09, 121.05 (CH).

### Beispiel 2:

25 g 6-Chlornicotinsäure und 14 g Natriumhydroxid werden in einem Gemisch aus 100 ml Wasser und 80 ml Methanol gelöst. Nach der Zugabe von 5 g Palladium (10 Gew.% auf Aktivkohle) als Katalysator wird 24 h bei 80-85 °C bei 0,1 MPa gerührt. Anschließend wird der Katalysator abfiltriert. Nach dem Ansäuern auf pH 1 mit Salzsäure fällt das Produkt, 2,2'-Bipyridyl-5,5'-dicarbonsäure, als weißer Feststoff aus. Man erhält 16,3 g (84 % Ausbeute). Das Rohprodukt hat nach ¹H NMR eine Reinheit von ca. 95 %.
¹H NMR (400 Mhz, DMSO): Entspricht Beispiel 1.

### Beispiel 3:

25 g 6-Chlornicotinsäure und 14 g Natriumhydroxid werden in einem Gemisch aus 100 ml Wasser und 80 ml Methanol gelöst. Nach der Zugabe von 10 g Palladium (5 Gew.% auf Aktivkohle) als Katalysator wird 30 h bei 80-85 °C bei 0,1 MPa gerührt. Anschließend wird der Katalysator abfiltriert. Nach dem Ansäuern auf pH 1 mit Salzsäure fällt das Produkt, 2,2'-Bipyridyl-5,5'-dicarbonsäure, als weißer Feststoff aus. Man erhält 15,3 g (79 % Ausbeute). Das Rohprodukt hat nach ¹H NMR eine Reinheit von ca. 95 %.
¹H NMR (400 Mhz, DMSO): Entspricht Beispiel 1.

### Beispiel 4:

9,50 g 2-Chlornicotinsäure und 5,30 g Natriumhydroxid werden in einem Gemisch aus 40 ml Wasser und 40 ml Methanol gelöst. Nach der Zugabe von 4 g Palladium (5 Gew.% auf Aktivkohle) als Katalysator wird 30 h bei 80-85 °C bei 0,1 MPa gerührt. Anschließend wird der Katalysator abfiltriert. Nach dem Ansäuern auf pH 1 mit Salzsäure fällt das Produkt, 2,2'-Bipyridyl-3,3'-dicarbonsäure, als weißer Feststoff aus. Man erhält 3,5 g (48 % Ausbeute). Das Rohprodukt hat nach ¹H NMR eine Reinheit von ca. 95 %. CAS Registry Number: 4433-01-6.
¹H NMR (360Mhz, DMSO): d = 13.6 (s breit, 2H); 8.51 (dd, J = 2, 5 Hz, 2 H) ; 8.17 (dd, J = 2, 8 Hz, 2 H) ; 7.49 (dd, J = 5, 8 Hz, 2 H).
¹³C NMR (90 Mhz, DMSO): d = 165.76, 157.53, 150.14 (CH), 138.08 (CH), 127.24, 121.00 (CH).

### Beispiel 5:

5,00 g 2-Chlorisonicotinsäure und 2,80 g Natriumhydroxid werden in einem Gemisch aus 20.ml Wasser und 16 ml Methanol gelöst. Nach der Zugabe von 1,02 g Palladium (10 Gew.% auf Aktivkohle) als Katalysator wird 6 h bei 80-85 °C bei 0,1 MPa gerührt. Anschließend wird der Katalysator abfiltriert. Nach dem Ansäuern auf pH 1 mit Salzsäure fällt das Produkt, 2,2'-Bipyridyl-4,4'-dicarbonsäure, als weißer Feststoff aus. Man erhält 3,3 g (85 % Ausbeute). Das Rohprodukt hat nach ¹H NMR eine Reinheit von ca. 90 %. CAS Registry Number: 6813-38-3.
¹H NMR (300Mhz, DMSO): d = 13.6 (s breit, 2H); 8.80 (m, 2 H) ; 8.31 (m, 2 H) ; 7.84 (m, 2 H).

### Beispiel 6:

2,55 g 6-Chlor-2-picolin und 1,00 g Natriumhydroxid werden in einem Gemisch aus 15 ml Wasser und 8 ml Methanol verrührt. Nach der Zugabe von 1,3 g Palladium (5 Gew.% auf Aktivkohle) als Katalysator wird 20 h bei 80-85 °C bei 0,1 MPa gerührt. Anschließend wird mit 20 ml Methanol verdünnt und der Katalysator abfiltriert. Nach dem Abziehen des Methanols kristallisiert das Produkt, 6,6'-Dimethyl-2,2'-bipyridyl, in Form blättriger Kristalle aus. Man erhält 1,10 g (58 % Ausbeute). Das Rohprodukt hat nach ¹H NMR eine Reinheit von> 98 %. CAS Registry Number: 4411-80-7.
¹H NMR (300Mhz, DMSO): d = 8.18 (d, J = 8 Hz, 2 H) ; 7.81 (t, J = 8 Hz, 2 H) ; 7.29 (d, J = 8 Hz, 2 H); 2.56 (s, 6H).

### Beispiel 7:

5,00 g 5-Chlor-2-hydroxypyridin und 4,00 g Natriumhydroxid werden in einem Gemisch aus 30 ml Wasser und 16 ml Methanol gelöst. Nach der Zugabe von 2,5 g Palladium (5 Gew.% auf Aktivkohle) als Katalysator wird 20 h bei 80-85 °C bei 0,1 MPa gerührt. Anschließend wird mit 20 ml Methanol verdünnt und der Katalysator abfiltriert. Nach dem Abziehen des Methanols wird mit Salzsäure auf pH 3 bis 4 angesäuert. Das Produkt, 6,6'-Dihydroxy-3,3'-bipyridyl (3,3'-Bipyridin-6,6'-Diol), fällt als weißer Feststoff aus. Man erhält 1,74 g (48 % Ausbeute). Das Rohprodukt hat nach ¹H NMR eine Reinheit von > 98 %. CAS Registry Number: 142929-10-0.
¹H NMR (360Mhz, DMSO): d = 11.38 (s breit, 2H); 7.61 (dd, J = 3, 10 Hz, 2 H) ; 7.50 (t, J = 3Hz, 2H); 6.37 (d, J = 3Hz, 2H).

### Beispiel 8:

5,00 g 6-Chlor-2-hydroxypyridin und 4,00 g Natriumhydroxid werden in einem Gemisch aus 30 ml Wasser und 16 ml Methanol gelöst. Nach der Zugabe von 2,5 g Palladium (5 Gew.% auf Aktivkohle) als Katalysator wird 20 h bei 80-85 °C bei 0,1 MPa gerührt. Anschließend wird mit 20 ml Methanol verdünnt und der Katalysator abfiltriert. Der Katalysator wird im Vakuum bei 80 °C getrocknet und in Beispiel 9 wieder eingesetzt. Nach dem Abziehen des Methanols wird mit Salzsäure auf pH 3 bis 4 angesäuert. Das Produkt, 6,6'-Dihydroxy-2,2'-bipyridyl (2,2'-Bipyridin-6,6'-Diol), fällt als weißer Feststoff aus. Man erhält 1,45 g (40 % Ausbeute). Das Rohprodukt hat nach ¹H NMR eine Reinheit von > 98 %. CAS Registry Number: 103505-54-0.
¹H NMR (360Mhz, DMSO): d = 10.74 (s breit, 2H); 7.63 (dd, J = 7, 9 Hz, 2 H) ; 7.21 (t, J = 7Hz, 2H); 6.56 (d, J = 9Hz, 2H).

### Beispiel 9 (Recyclisierung des Katalysators):

5,00 g 6-Chlor-2-hydroxypyridin und 4,00 g Natriumhydroxid werden in einem Gemisch aus 30 ml Wasser und 16 ml Methanol gelöst. Nach der Zugabe des in Beispiel 8 abgetrennten Katalysators wird 20 h bei 80-85 °C bei 0,1 MPa gerührt. Anschließend wird mit 20 ml Methanol verdünnt und der Katalysator abfiltriert. Nach dem Abziehen des Methanols wird mit Salzsäure auf pH 3 bis 4 angesäuert. Das Produkt, 6,6'-Dihydroxy-2,2'-bipyridyl (2,2'-Bipyridin-6,6'-Diol), fällt als weißer Feststoff aus. Man erhält 2,2 g (61 % Ausbeute). Das Rohprodukt hat nach ¹H NMR eine Reinheit von > 98 %.
¹H NMR (360Mhz, DMSO): Entspricht Beispiel 7.

### Beispiel 10:

5,00 g 2-Chlor-Chinolin und 1,80 g Natriumhydroxid werden in einem Gemisch aus 20 ml Wasser und 16 ml Methanol gelöst. Nach der Zugabe von 1,5 g Palladium (10 Gew.% auf Aktivkohle) als Katalysator wird 24 h bei 80-85 °C bei 0,1 MPa gerührt. Anschließend wird nach dem Abkühlen der Katalysator und das ausgefallene Produkt abfiltriert. Durch Extraction des Katalysators erhält man 1,7 g 2,2'- Bichinolin (43 % Ausbeute). Das Rohprodukt hat nach ¹H NMR eine Reinheit von ca. 98 %. CAS Registry Number: 119-91-5.
¹H NMR (300Mhz, DMSO): d = 8.81 (d, J = 9 Hz, 2H); 8.58 (d, J = 9 Hz, 2 H): 8.20 (d breit, J = 9 Hz, 2 H); 8.08 (d breit, J = 8 Hz, 2 H); 7.86 (ddd, J = 2, 7, 9 Hz, 2 H), 7.86 (ddd, J = 1, 7, 8 Hz, 2 H).

## Patentansprüche

1. Verfahren zur Herstellung von Bipyridylen durch Umsetzung von mindestens einem Pyridin der allgemeinen Formel (III) 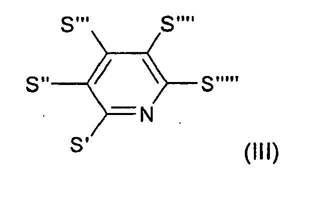 worin
S', S", S"', S"" und S""' unabhängig voneinander gleich oder verschieden Wasserstoff, (C₁-C₁₈)-Alkyl, Alkoxy-(C₁-C₁₈), Acyloxy-(C₁-C₁₈), Aryloxy-, Perfluoroacyloxy-(C₁-C₈), NO₂, Aryl, Heteroaryl, Halogen, Hydroxy, Nitroso, CN, COOH, CHO, PO₃H₂, SO₃H, SO₂R, SOR, NH₂, NH-Alkyl-(C₁-C₁₈), N-Alkyl₂-(C₁-C₁₈), geschütztes Amin, CF₃, NHCO-Alkyl-(C₁-C₄), N-Alkyl-(C₁-C₄)-CO-Alkyl-(C₁-C₄), COO-Alkyl-(C₁-C,₈), CONH₂, CO-Alkyl-(C₁-C₁₈), NHCOH, NHCOO-Alkyl-(C₁-C₄), CO-Aryl, COO-Aryl, CHCH-CO₂-Alkyl-(C₁-C₁₈), CHCHCO₂H, PO-Phenyl₂, PO-Alkyl₂₋(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und
(O⁻)ₙ(Kation)ⁿ⁺ bedeuten,
wobei Kation Alkali, Erdalkali, NR₂H₂, NR₃H, NRH₃, NR₄, NH₄, PR₂H₂, PR₃H, PRH₃, PR₄ oder/und PH₄ und Aryl, Aryloxy und Heteroaryl Reste mit bis zu 18C-Atomen bedeutet
wobei R Aryl mit bis zu 18 C-Atomen oder/und (C₁-C₁₈)-Alkyl, unabhängig voneinander gleich oder verschieden, bedeutet,
und wobei mindestens einer der Substituenten von S', S", S"', S"" und S""' ein nicht inerter Substituent wie OH, COOH, PO₃H₂ oder SO₃H, (COO⁻)ₙ(Kation)ⁿ⁺,
(PO₃²⁻)ₙ(Kation)₂ⁿ⁺, (SO₃⁻)ₙ(Kation)ⁿ⁺ oder/und (O⁻)ₙ(Kation)ⁿ⁺ ist,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet,
und wobei mindestens einer der Substituenten von S', S", S"', S"" und S""' Chlorid, Bromid oder Jodid ist,
wobei gegebenenfalls S', S", S"', S"" oder/und S""' untereinander zusammen einen oder mehrere aliphatische oder/und aromatische Ringe bilden
oder/und worin gegebenenfalls S', S", S"', S"" oder/und S""' eine Brücke zu mindestens einem weiteren Pyridin der allgemeinen Formel (III) bildet
oder/und worin gegebenenfalls die Reste S', S", S"', S"" oder/und S""' die oben angegebene Bedeutung besitzen und mit mindestens einem Rest, der die oben angegebene Bedeutung für S', S", S"', S"" oder/und S""' besitzt, substituiert ist
in Wasser in Gegenwart eines Alkohols, einer Base, eines Palladium-Katalysators und gegebenenfalls eines oder mehrerer weiterer Lösemittel bei einer Temperatur von 0 - 200 °C.

2. Verfahren nach Anspruch 1, **dadurch gekennzeichnet, daß** S', S", S"', S"" und S""' unabhängig voneinander, gleich oder verschieden, Wasserstoff, Methyl, Ethyl, Isopropyl, Methoxy, Acetoxy, Trifluoracetoxy, Trifluormethyl, O-Phenyl, Phenyl, Fluor, OH, Nitroso, NO₂, CN, COOH, CHO, PO₃H₂, SO₃H, NH₂, NH-Alkyl-(C₁-C₈), N-Alkyl₂-(C₁-C₈), NHCO-Alkyl-(C₁-C₄), N-Alkyl-(C₁-C₄)-CO-Alkyl-(C₁-C₄), CONH₂, CO-Alkyl-(C₁-C₈), NHCOH, CO-Phenyl, CHCHCO₂H, PO-Phenyl₂, PO-Alkyl₂-(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und
(O⁻)ₙ(Kation)ⁿ⁺ bedeuten,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet.

3. Verfahren nach Anspruch 1, **dadurch gekennzeichnet, daß** zur Herstellung von symmetrischen 2,2'-Bipyridylen der allgemeinen Formel (IV), symmetrischen 3,3'-Bipyridylen der allgemeinen Formel (V) oder symmetrischen 4,4'-Bipyridylen der allgemeinen Formel (V1), 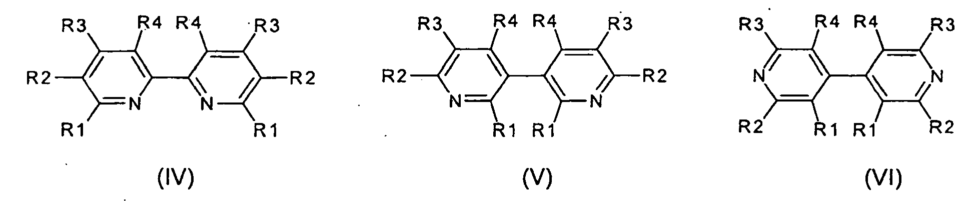 worin
R1 bis R4 unabhängig voneinander, gleich oder verschieden, Wasserstoff, (C₁-C₁₈)-Alkyl, Alkoxy-(C₁-C₁₈), Acyloxy-(C₁-C₁₈), Aryloxy, Aryl, Heteroaryl, Fluor, Hydroxy, Nitro, Nitroso, CN, COOH, CHO, PO₃H₂, SO₃H, SO₂R, SOR, NH₂, NH-Alkyl-(C₁-C₁₈), N-Alkyl₂-(C₁-C₁₈), geschütztes Amin, CF₃, NHCO-Alkyl-(C₁-C₄), N-Alkyl-(C₁-C₄)-CO-Alkyl-(C₁-C₄), COO-Alkyl-(C₁-C₁₈), CONH₂, CO-Alkyl-(C₁-C₁₈), NHCOH, NHCOO-Alkyl-(C₁-C₄), CO-Aryl, COO-Aryl, CHCH-CO₂-Alkyl-(C₁-C₁₈), CHCHCO₂H, PO-Phenyl₂, PO-Alkyl₂-(C₁-C₄), (COO⁻)ₙ(Kation)ⁿ⁺,
(PO₃²⁻)ₙ(Kation)₂ⁿ⁺, (SO₃⁻)ₙ(Kation)ⁿ⁺ oder/und (O⁻)ₙ(Kation)ⁿ⁺ bedeuten,
wobei Kation Erdalkali, Alkali, NR₂H₂, NR₃H, NRH₃, NR₄, NH₄, PR₂H₂, PR₃H, PRH₃, PR₄ oder/und PH₄ und Aryl, Aryloxy und Heteroaryl Rest mit bis zu 18 C-Atomen bedeutet,
wobei R Aryl mit bis zu 18 C-Atomen oder/und (C₁-C₁₈)-Alkyl unabhängig voneinander gleich oder verschieden bedeutet,
wobei gegebenenfalls R1 bis R4 untereinander zusammen einen oder mehrere aliphatische oder/und aromatische Ringe bilden
und wobei mindestens einer der Substituenten von R1 bis R4 ein nicht inerter Substituent wie Acyloxy-(C₁-C₈), Hydroxy, COOH, PO₃H₂, SO₃H, SO₂R, NH₂, NH-Alkyl-(C₁-C₈), N-Alkyl₂-(C₁-C₈), geschütztes nicht inertes Amin, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), COO-Aryl mit bis zu 18 C-Atomen, CHCH-CO₂₋Alkyl-(C₁-C₈), CHCHCO₂H, (COO⁻)ₙ(Kation)ⁿ⁺, (PO₃²⁻)ₙ(Kation)₂ⁿ⁺, (SO₃⁻)ₙ(Kation)ⁿ⁺ oder/und
(O⁻)ₙ(Kation)ⁿ⁺ ist,
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist,
2-Halogenpyridine der allgemeinen Formel (VII), 3-Halogenpyridine der allgemeinen Formel (VIII) oder 4-Halogenpyridine der allgemeinen Formel (IX) umgesetzt werden, 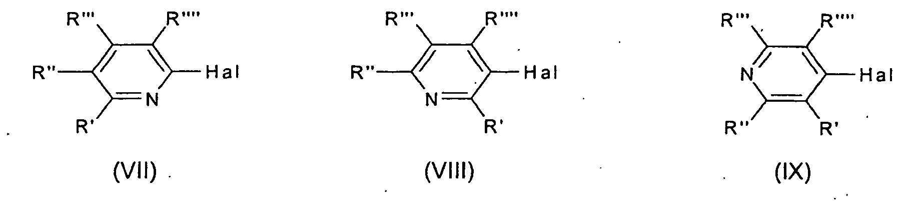 worin R', R", R'" und R"" die oben angegebene Bedeutung für R1 bis R4 besitzen und worin Hal Chlor, Brom oder lod bedeutet,
in Wasser in Gegenwart eines Alkohols, einer Base, eines Palladium-Katalysators und gegebenenfalls eines oder mehrerer weiterer Lösemittel bei einer Temperatur von 0 - 200 °C.

4. Verfahren nach Anspruch 3, **dadurch gekennzeichnet, daß** R1 bis R4 unabhängig voneinander, gleich oder verschieden, bedeuten: Wasserstoff, C₁-C₈₋Alkyl, Alkoxy-(C₁-C₈), Acyloxy-(C₁-C₈), Aryloxy, Aryl, Heteroaryl, Fluor, Hydroxy, Nitro, Nitroso, CN, COOH, CHO, PO₃H₂, SO₃H, SO₂R, SOR, NH₂, NH-Alkyl-(C₁-C₈), N-Alkyl₂-(C₁-C₈), geschütztes Amin, CF₃, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), CO-Aryl, COO-Aryl, CHCH-CO₂-Alkyl-(C₁-C₈), CHCHCO₂H, PO-Phenyl₂, PO-Alkyl₂-(C₁-C₄),
(COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺,
wobei Kation Na, Li, K, Ca, Mg, NR₃H, NR₄, NH₄, PR₃H, PR₄ oder/und PH₄ und Aryl, Aryloxy und Heteroaryl mit bis zu 18 C-Atomen bedeutet und wobei R Aryl mit bis zu 18 C-Atomen oder/und (C₁-C₁₈)-Alkyl unabhängig voneinander gleich oder verschieden bedeutet
und wobei mindestens einer der Substituenten von R1 bis R4 ein nicht inerter Substituent wie Acyloxy-(C₁-C₈), Hydroxy, COOH, PO₃H₂, SO₃H, SO₂R, NH₂, NH-Alkyl-(C₁-C₈), N-Alkyl₂-(C₁-C₈), geschütztes nicht inertes Amin, NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), COO-Aryl mit bis zu 18 C-Atomen, CHCH-CO₂₋Alkyl-(C₁-C₈), CHCHCO₂H, (COO⁻)ₙ(Kation)ⁿ⁺, (PO₃²⁻)ₙ(Kation)₂ⁿ⁺, (SO₃⁻)ₙ(Kation)ⁿ⁺ oder/und
(O⁻)ₙ(Kation)ⁿ⁺ ist,
wobei Kation Na, Li, K, Ca, Mg, NR₃H, NR₄, NH₄, PR₃H, PR₄ oder/und PH₄ ist und
wobei R Aryl mit bis zu 18 C-Atomen oder/und (C₁-C₁₈)-Alkyl unabhängig voneinander gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

5. Verfahren nach Anspruch 3, **dadurch gekennzeichnet, daß** R1 bis R4 unabhängig voneinander, gleich oder verschieden, bedeuten: Wasserstoff, Methyl, Ethyl, Tert.-Butyl, Isopropyl, Methoxy, Acetoxy, Trifluoracetoxy, Trifluormethyl, O-Phenyl, Phenyl, Fluor, OH, Nitroso, NO₂, CN, COOH, CHO, PO₃H₂, SO₃H, NH₂, NH-Alkyl (C₁-C₈), N-Alkyl₂-(C₁-C₈), NHCO-Alkyl-(C₁-C₄), N-Alkyl-(C₁-C₄)-CO-Alkyl-(C₁₋C₄), COO-Alkyl-(C₁-C₈), CONH₂, CO-Alkyl-(C₁-C₈), NHCOH, NHCOO-Alkyl-(C₁-C₄), CO-Phenyl, COO-Phenyl, CHCH-CO₂-Alkyl-(C₁-C₈), CHCHCO₂H, PO-Phenyl₂, PO-Alkyl₂-(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺,
wobei Kation Na, Li, K, NR₃H, NR₄, PR₃H oder/und PR₄ und
wobei R Aryl mit bis zu 18 C-Atomen oder/und (C₁-C₁₈)-Alkyl unabhängig voneinander gleich oder verschieden bedeutet
und wobei mindestens einer der Substituenten von R1 bis R4 ein nicht inerter Substituent wie Acetoxy, Trifluoracetoxy, OH, COOH, PO₃H₂, SO₃H, NH₂, NH-Alkyl-(C₁-C₈), N-Alkyl₂-(C₁-C₈), NHCO-Alkyl-(C₁-C₄), N-Alkyl-(C₁-C₄)-CO-Alkyl-(C₁-C₄), COO-Alkyl-(C₁-C₈), CONH₂, CO-Alkyl-(C₁-C₈), NHCOH, NHCOO-Alkyl-(C₁-C₄), COO-Phenyl, CHCH-CO₂-Alkyl-(C₁-C₈), CHCHCO₂H,
(COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺ ist,
wobei Kation Na, Li, K, NR₃H, NR₄, PR₃H oder/und PR₄ und
wobei R (C₆-C₁₈)-Aryl oder/und (C₁-C₁₈)-Alkyl gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

6. Verfahren nach Anspruch 3, **dadurch gekennzeichnet, daß** R1 bis R4 unabhängig voneinander, gleich oder verschieden, bedeuten: Wasserstoff, Methyl, Ethyl, Isopropyl, Methoxy, Acetoxy, Trifluoracetoxy, Trifluormethyl, Trichlormethyl, O-Phenyl, Phenyl, Fluor, OH, Nitroso, NO₂, CN, COOH, CHO, PO₃H₂, SO₃H, NH₂, NH-Alkyl-(C₁-C₈), N-Alkyl₂-(C₁-C₈), NHCO-Alkyl-(C₁-C₄), N-Alkyl-(C₁-C₄)-CO-Alkyl-(C₁₋C₄), CONH₂, CO-Alkyl-(C₁-C₈), NHCOH, CO-Phenyl, CHCHCO₂H, PO-Phenyl₂, PO-Alkyl₂-(C₁-C₄), (COO⁻)ₙ(Kation)ⁿ⁺, (PO₃²⁻)ₙ(Kation)₂ⁿ⁺, (SO₃⁻)ₙ(Kation)ⁿ⁺ oder/und (O⁻)ₙ(Kation)ⁿ⁺,
wobei Kation Na, K, NR₄ oder/und PR₄ und
wobei R Aryl mit bis zu 18 C-Atomen oder/und (C₁-C₁₈)-Alkyl unabhängig voneinander gleich oder verschieden bedeutet
und wobei mindestens einer der Substituenten von R1 bis R4 ein nicht inerter Substituent wie OH, COOH, PO₃H₂ oder SO₃H, (COO⁻)ₙ(Kation)ⁿ⁺,
(PO₃²⁻)n(Kation)₂ⁿ⁺, (SO₃⁻)ₙ(Kation)ⁿ⁺ oder/und (O⁻)ₙ(Kation)ⁿ⁺ ist,
wobei Kation Na, K, NR₄ oder/und PR₄ und
wobei R Aryl mit bis zu 18 C-Atomen oder/und (C₁-C₁₈)-Alkyl gleich oder verschieden bedeutet,
wobei gegebenenfalls R1 bis R4 untereinander zusammen einen oder mehrere aliphatische oder/und aromatische Ringe bilden
oder/und worin gegebenenfalls R1, R2, R3 oder R4 des einen Ringes eine Brücke zu R1, R2, R3 oder R4 des zweiten Ringes bildet
oder/und worin gegebenenfalls die Reste R1 bis R4 die oben angegebene Bedeutung besitzen und mit mindestens einem Rest, der die oben angegebene Bedeutung für R1 bis R4 besitzt, substituiert ist.

7. Verfahren nach einem der Ansprüche 1 bis 6, **dadurch gekennzeichnet, daß** die Salze der Halogenpyridine für die Umsetzung verwendet werden.

8. Verfahren nach einem der Ansprüche 1 bis 7, **dadurch gekennzeichnet, daß** die Reste R', R", R'" oder/und R"" substituiert sind.

9. Verfahren nach Anspruch 8, **dadurch gekennzeichnet, daß** die Reste R', R", R'" oder/und R"" durch die Reste R1 bis R4 substituiert sind.

10. Verfahren nach einem der Ansprüche 1 bis 8, **dadurch gekennzeichnet, daß** beim Einsatz von zwei oder mehr verschiedenen Monohalogenpyridinen neben den symmetrischen Bipyridylen auch unsymmetrisch gekoppelte Bipyridyle erzeugt werden.

11. Verfahren nach einem der Ansprüche 1 bis 10, **dadurch gekennzeichnet, daß** beim Einsatz von Halogenpyridylen ein Wasseranteil im Reaktionsmedium von mindestens 35 % verwendet wird.

12. Verfahren nach einem der Ansprüche 1 bis 11, **dadurch gekennzeichnet, daß** als Palladium-Katalysator ein trägerloses oder/und geträgertes Palladium verwendet wird, wobei ein metallisches Palladium, als auch eine Palladium-Verbindung, gegebenenfalls in Kombination miteinander, verwendet werden kann.

13. Verfahren nach Anspruch 12, **dadurch gekennzeichnet, daß** als Träger für Palladium Aktivkohle, Metalloxide oder/und Metallsalze eingesetzt werden.

14. Verfahren nach Anspruch 12 oder 13, **dadurch gekennzeichnet, daß** die Beladung des Palladiums auf dem Träger mindestens 5 Gew.-% beträgt.

15. Verfahren nach einem der Ansprüche 12 bis 14, **dadurch gekennzeichnet, daß** der Palladium-Katalysator, jeweils berechnet auf Palladiummetall, in einer Menge von 0,0001 bis 10 mol%, in Bezug auf Halogenpyridin eingesetzt wird.

16. Verfahren nach einem der Ansprüche 1 bis 15, **dadurch gekennzeichnet, daß** als Alkohol solche, die nach der Oxidation zum Aldehyd bzw. Keton keine bzw. nur schwer eine Aldolreaktion eingehen können, verwendet werden.

17. Verfahren nach einem der Ansprüche 1 bis 16, **dadurch gekennzeichnet, daß** als Base Alkali- oder/und Erdalkalisalze eingesetzt werden.

18. Verfahren nach einem der Ansprüche 1 bis 17, **dadurch gekennzeichnet, daß** die Reaktion bei einer Temperatur von 0 bis 200 °C und bei einem Druck von 0,1 bis 2 MPa durchgeführt wird.

19. Verfahren nach einem der Ansprüche 1 bis 18, **dadurch gekennzeichnet, daß** das Reaktionsprodukt 2,2'-Bipyridyl-4,4'-dicarbonsäure enthält.

20. Verfahren nach einem der Ansprüche 1 bis 19, **dadurch gekennzeichnet, daß** das Reaktionsprodukt mit einem Übergangsmetall der 7. oder/und 8. Nebengruppe oder/und mindestens einem Edelmetall-zu mindestens einem Bipyridyl-Metallkomplex umgesetzt wird.

21. Verwendung eines Reaktionsproduktes hergestellt nach einem Verfahren nach Anspruch 20 in photovoltaischen oder photoelektrochemischen Zellen oder für die photoinduzierte Elektrolyse.

## Claims

1. Process for the preparation of bipyridyls by reaction of at least one pyridine of the general formula (III) 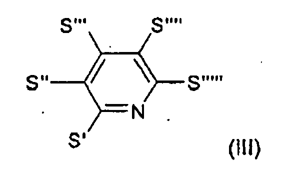 in which
S', S", S"', S"" and S""', independently of one another, identically or differently, denote hydrogen, (C₁-C₁₈)-alkyl, alkoxy-(C₁-C₁₈), acyloxy-(C₁-C₁₈), aryloxy, perfluoroacyloxy-(C₁-C₈), NO₂, aryl, heteroaryl, halogen, hydroxyl, nitroso, CN, COOH, CHO, PO₃H₂, SO₃H, SO₂R, SOR, NH₂, NH-alkyl-(C₁-C₁₈), N-alkyl₂-(C₁-C₁₈), protected amine, CF₃, NHCO-alkyl-(C₁-C₄), N-alkyl-(C₁-C₄)-CO-alkyl-(C₁-C₄), COO-alkyl-(C₁-C₁₈), CONH₂, CO-alkyl-(C₁-C₁₈), NHCOH, NHCOO-alkyl-(C₁-C₄), CO-aryl, COO-aryl, CHCH-CO₂-alkyl-(C₁-C₁₈), CHCHCO₂H, PO-phenyl₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ or/and
(O⁻)ₙ(cation)ⁿ⁺,
where cation denotes alkali metal, alkaline earth metal, NR₂H₂, NR₃H, NRH₃, NR₄, NH₄, PR₂H₂ , PR₃H, PRH₃, PR₄ or/and PH₄ and aryl, aryloxy and heteroaryl denote radicals having up to 18 C atoms,
where R, independently of one another, identically or differently, denotes aryl having up to 18 C atoms or/and (C₁-C₁₈)-alkyl,
and where at least one of the substituents of S', S", S"', S"" and S""' is a non-inert substituent, such as OH, COOH, PO₃H₂ or SO₃H, (COO⁻)ₙ₋(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes Na, K, NR₄ or/and PR₄ and
where R, independently of one another, identically or differently, denotes aryl having up to 18 C atoms or/and (C₁-C₁₈)-alkyl,
and where at least one of the substituents of S', S", S"', S"" and S""' is chloride, bromide or iodide,
where S', S", S"', S"" or/and S""' together with one another optionally form one or more aliphatic or/and aromatic rings
or/and in which S', S", S"', S"" or/and S""' optionally forms a bridge to at least one further pyridine of the general formula (III)
or/and in which the radicals S', S", S"', S"" or/and S""' optionally have the above-mentioned meaning and are substituted by at least one radical which has the above-mentioned meaning for S', S", S"', S"" or/and S""', in water in the presence of an alcohol, a base, a palladium catalyst and optionally one or more further solvents at a temperature of 0 - 200°C.

2. Process according to Claim 1, **characterised in that** S', S", S"', S"" and S""', independently of one another, identically or differently, denote hydrogen, methyl, ethyl, isopropyl, methoxy, acetoxy, trifluoroacetoxy, trifluoromethyl, O-phenyl, phenyl, fluorine, OH, nitroso, NO₂, CN, COOH, CHO, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), CONH₂, CO-alkyl-(C₁-C₈), NHCOH, CO-phenyl, CHCHCO₂H, PO-phenyl₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ or/and
(O⁻)ₙ(cation)ⁿ⁺,
where cation denotes Na, K, NR₄ or/and PR₄ and
where R, independently of one another, identically or differently, denotes aryl having up to 18 C atoms or/and (C₁-C₁₈)-alkyl.

3. Process according to Claim 1, **characterised in that**, for the preparation of symmetrical 2,2'-bipyridyls of the general formula (IV), symmetrical 3,3'-bipyridyls of the general formula (V) or symmetrical 4,4'-bipyridyls of the general formula (VI) 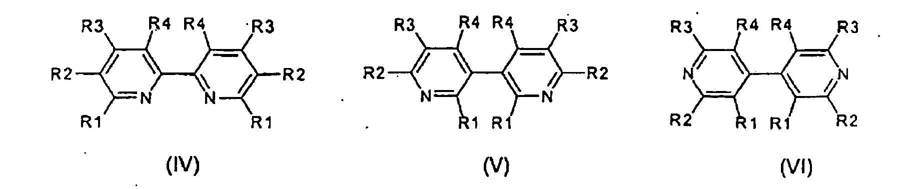 in which
R1 to R4, independently of one another, identically or differently, denote hydrogen, (C₁-C₁₈)-alkyl, alkoxy-(C₁-C₁₈), acyloxy-(C₁-C₁₈), aryloxy, aryl, heteroaryl, fluorine, hydroxyl, nitro, nitroso, CN, COOH, CHO, PO₃H₂, SO₃H, SO₂R, SOR, NH₂, NH-alkyl-(C₁-C₁₈), N-alkyl₂-(C₁-C₁₈), protected amine, CF₃, NHCO-alkyl-(C₁-C₄), N-alkyl-(C₁-C₄)-CO-alkyl-(C₁-C₄), COO-alkyl-(C₁-C₁₈), CONH₂, CO-alkyl-(C₁-C₁₈), NHCOH, NHCOO-alkyl-(C₁-C₄), CO-aryl, COO-aryl, CHCH-CO₂-alkyl-(C₁-C₁₈), CHCHCO₂H, PO-phenyl₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes alkaline earth metal, alkali metal, NR₂H₂, NR₃H, NRH₃, NR₄, NH₄, PR₂H₂, PR₃H, PRH₃, PR₄ or/and PH₄, and aryl, aryloxy and heteroaryl denote radicals having up to 18 C atoms,
where R, independently of one another, identically or differently, denotes aryl having up to 18 C atoms or/and (C₁-C₁₈)-alkyl,
where R1 to R4 together with one another optionally form one or more aliphatic or/and aromatic rings
and where at least one of the substituents of R1 to R4 is a non-inert substituent, such as acyloxy-(C₁-C₈), hydroxyl, COOH, PO₃H₂, SO₃H, SO₂R, NH₂, NH-alkyl-(C₁-C₈), N-alkyl₂-(C₁-C₈), protected non-inert amine, NHCO-alkyt-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-aryl having up to 18 C atoms, CHCH-CO₂-alkyl-(C₁-C₈), CHCHCO₂H, (COO⁻)ₙ(cation)ⁿ⁺, (PO₃²⁻)ₙ(catlon)₂ⁿ⁺, (SO₃⁻)ₙ(cation)ⁿ⁺ or/and (O⁻)ₙ(cation)ⁿ⁺,
or/and in which R1, R2, R3 or R4 of the first ring optionally forms a bridge to R1, R2, R3 or R4 of the second ring
or/and in which the radicals R1 to R4 optionally have the above-mentioned meaning and are substituted by at least one radical which has the above-mentioned meaning for R1 to R4,
2-halopyridines of the general formula (VII), 3-halopyridines of the general formula (VIII) or 4-halopyridines of the general formula (IX) 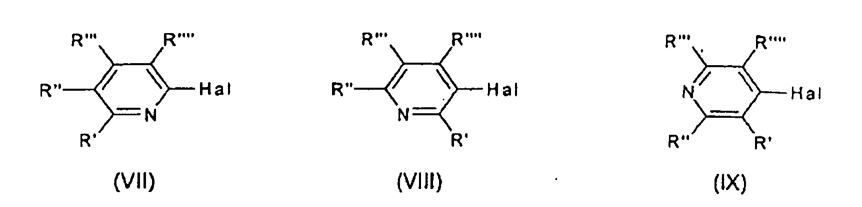 in which R', R", R"' and R"" have the above-mentioned meaning for R1 to R4 and in which Hal denotes chlorine, bromine or iodine,
are reacted in water in the presence of an alcohol, a base, a palladium catalyst and optionally one or more further solvents at a temperature of 0 - 200°C.

4. Process according to Claim 3, **characterised in that** R1 to R4, independently of one another, identically or differently, denote: hydrogen, C₁-C₈-alkyl, alkoxy-(C₁-C₈), acyloxy-(C₁-C₈), aryloxy, aryl, heteroaryl, fluorine, hydroxyl, nitro, nitroso, CN, COOH, CHO, PO₃H₂, SO₃H, SO₂R, SOR, NH₂, NH-alkyl-(C₁-C₈), N-alkyl₂-(C₁-C₈), protected amine, CF₃, NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), CO-aryl, COO-aryl, CHCH-CO₂-alkyl-(C₁-C₈), CHCHCO₂H, PO-phenyl₂, PO-alkyl₂-(C₁-C₄),
(COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ₋(cation)ⁿ⁺,
where cation denotes Na, Li, K, Ca, Mg, NR₃H, NR₄, NH₄, PR₃H, PR₄ or/and PH₄ and aryl, aryloxy and heteroaryl denote radicals having up to 18 C atoms and where R, independently of one another, identically or differently, denotes aryl having up to 18 C atoms or/and (C₁-C₁₈)-alkyl, and where at least one of the substituents of R1 to R4 is a non-inert substituent, such as acyloxy-(C₁-C₈), hydroxyl, COOH, PO₃H₂, SO₃H, SO₂R, NH₂, NH-alkyl-(C₁-C₈), N-alkyl₂-(C₁-C₈), protected non-inert amine, NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-aryl having up to 18 C atoms, CHCH-CO₂-alkyl-(C₁-C₈), CHCHCO₂H, (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes Na, Li, K, Ca, Mg, NR₃H, NR₄, NH₄, PR₃H, PR₄ or/and PH₄, and where R, independently of one another, identically or differently, denotes aryl having up to 18 C atoms or/and (C₁-C₁₈)-alkyl,
where R1 to R4 together with one another optionally form one or more aliphatic or/and aromatic rings
or/and in which R1, R2, R3 or R4 of the first ring optionally forms a bridge to R1, R2, R3 or R4 of the second ring
or/and in which the radicals R1 to R4 optionally have the above-mentioned meaning and are substituted by at least one radical which has the above-mentioned meaning for R1 to R4.

5. Process according to Claim 3, **characterised in that** R1 to R4, independently of one another, identically or differently, denote: hydrogen, methyl, ethyl, tert-butyl, isopropyl, methoxy, acetoxy, trifluoroacetoxy, trifluoromethyl, O-phenyl, phenyl, fluorine, OH, nitroso, NO₂, CN, COOH, CHO, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), CO-phenyl, COO-phenyl, CHCH-CO₂-alkyl-(C₁-C₈), CHCHCO₂H, PO-phenyl₂, PO-alkyl₂₋(C₁-C₄), (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes Na, Li, K, NR₃H, NR₄, PR₃H or/and PR₄ and
where R, independently of one another, identically or differently, denotes aryl having up to 18 C atoms or/and (C₁-C₁₈)-alkyl,
and where at least one of the substituents of R1 to R4 is a non-inert substituent, such as acetoxy, trifluoroacetoxy, OH, COOH, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-phenyl, CHCH-CO₂-alkyl-(C₁-C₈), CHCHCO₂H, (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes Na, Li, K, NR₃H, NR₄, PR₃H or/and PR₄ and
where R denotes (C₆-C₁₈)-aryl or/and (C₁-C₁₈)-alkyl, identically or differently,
where R1 to R4 together with one another optionally form one or more aliphatic or/and aromatic rings
or/and in which R1, R2, R3 or R4 of the first ring optionally forms a bridge to R1, R2, R3 or R4 of the second ring
or/and in which the radicals R1 to R4 optionally have the above-mentioned meaning and are substituted by at least one radical which has the above-mentioned meaning for R1 to R4.

6. Process according to Claim 3, **characterised in that** R1 to R4, independently of one another, identically or differently, denote: hydrogen, methyl, ethyl, isopropyl, methoxy, acetoxy, trifluoroacetoxy, trifluoromethyl, trichloromethyl, O-phenyl, phenyl, fluorine, OH, nitroso, NO₂, CN, COOH, CHO, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), CONH₂, CO-alkyl-(C₁-C₈), NHCOH, CO-phenyl, CHCHCO₂H, PO-phenyl₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ₋(cation)ⁿ⁺,
where cation denotes Na, K, NR₄ or/and PR₄ and
where R, independently of one another, identically or differently, denotes aryl having up to 18 C atoms or/and (C₁-C₁₈)-alkyl,
and where at least one of the substituents of R1 to R4 is a non-inert substituent, such as OH, COOH, PO₃H₂ or SO₃H, (COO⁻)ₙ(cation)ⁿ⁺, (PO₃²⁻)ₙ(cation)₂ⁿ⁺, (SO₃⁻)ₙ(cation)ⁿ⁺ or/and (O⁻)ₙ(cation)ⁿ⁺,
where cation denotes Na, K, NR₄ or/and PR₄ and
where R denotes aryl having up to 18 C atoms or/and (C₁-C₁₈)-alkyl, identically or differently,
where R1 to R4 together with one another optionally form one or more aliphatic or/and aromatic rings
or/and in which R1, R2, R3 or R4 of the first ring optionally forms a bridge to R1, R2, R3 or R4 of the second ring
or/and in which the radicals R1 to R4 optionally have the above-mentioned meaning and are substituted by at least one radical which has the above-mentioned meaning for R1 to R4.

7. Process according to one of Claims 1 to 6, **characterised in that** the salts of the halopyridines are used for the reaction.

8. Process according to one of Claims 1 to 7, **characterised in that** the radicals R', R", R'" or/and R"" are substituted.

9. Process according to Claim 8, **characterised in that** the radicals R', R", R"' or/and R"" are substituted by the radicals R1 to R4.

10. Process according to one of Claims 1 to 8, **characterised in that**, on use of two or more different monohalopyridines, asymmetrically coupled bipyridyls are also produced in addition to the symmetrical bipyridyls.

11. Process according to one of Claims 1 to 10, **characterised in that**, on use of halopyridyls, a proportion of water in the reaction medium of at least 35% is used.

12. Process according to one of Claims 1 to 11, **characterised in that** the palladium catalyst used is an unsupported or/and supported palladium, where a metallic palladium and also a palladium compound, optionally in combination with one another, can be used.

13. Process according to Claim 12, **characterised in that** the supports employed for palladium are activated carbon, metal oxides or/and metal salts.

14. Process according to Claim 12 or 13, **characterised in that** the loading of the palladium on the support is at least 5% by weight.

15. Process according to one of Claims 12 to 14, **characterised in that** the palladium catalyst, in each case calculated on the basis of palladium metal, is employed in an amount of 0.0001 to 10 mol%, based on halopyridine.

16. Process according to one of Claims 1 to 15, **characterised in that** the alcohol used is only able to undergo an aldol reaction with difficulty, or not at all, after oxidation to the aldehyde or ketone.

17. Process according to one of Claims 1 to 16, **characterised in that** the base employed is alkali metal or/and alkaline earth metal salts.

18. Process according to one of Claims 1 to 17, **characterised in that** the reaction is carried out at a temperature of 0 to 200°C and at a pressure of 0.1 to 2 MPa.

19. Process according to one of Claims 1 to 18, **characterised in that** the reaction product comprises 2,2'-bipyridyl-4,4'-dicarboxylic acid.

20. Process according to one of Claims 1 to 19, **characterised in that** the reaction product is reacted with a transition metal from sub-group 7 or/and 8 or/and at least one noble metal to give at least one bipyridyl/metal complex.

21. Use of a reaction product produced by a process according to Claim 20 in photovoltaic or photoelectrochemical cells or for photoinduced electrolysis.

## Revendications

1. Procédé pour la préparation de bipyridyles par réaction d'au moins une pyridine de la formule générale (III) 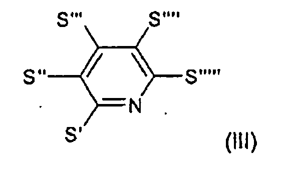 dans laquelle
S', S", S"', S"" et S""', indépendamment les uns des autres, de manière identique ou différente, représentent hydrogène, (C₁-C₁₈)-alkyle, alkoxy-(C₁-C₁₈), acyloxy-(C₁-C₁₈), aryloxy, perfluoroacyloxy-(C₁-C₈), NO₂, aryle, hétéroaryle, halogène, hydroxyle, nitroso, CN, COOH, CHO, PO₃H₂, SO₃H, SO₂R, SOR, NH₂, NH-alkyl-(C₁-C₁₈), N-alkyl₂-(C₁-C₁₈), amine protégée, CF₃, NHCO-alkyl-(C₁-C₄), N-alkyl-(C₁-C₄)-CO-alkyl-(C₁-C₄), COO-alkyl-(C₁-C₁₈), CONH₂, CO-alkyl-(C₁-C₁₈), NHCOH, NHCOO-alkyl-(C₁-C₄), CO-aryle, COO-aryle, CHCH-CO₂-alkyl-(C₁-C₁₈), CHCHCO₂H, PO-phényle₂, PO-alkyle₂-(C₁-C₄), (COO⁻)ₙ(cation)ⁿ⁺ (PO₃²⁻)ₙ(cation)₂ⁿ+, (SO3⁻)ₙ(cation)ⁿ⁺ ou/et (O⁻)ₙ(cation)ⁿ⁺,
où cation représente un métal alcalin, un métal alcalino-terreux, NR₂H₂, NR₃H, NRH₃, NR₄, NH₄, PR₂H₂, PR₃H, PRH₃, PR₄ ou/et PH₄ et aryle, aryloxy et hétéroaryle représentent des radicaux comportant jusqu'à 18 atomes de C,
où R, indépendamment les uns des autres, de manière identique ou différente, représente aryle comportant jusqu'à 18 atomes de C ou/et (C₁-C₁₈)-alkyle,
et où au moins l'un des substituants de S', S", S"', S"" et S""' est un substituant non inerte, tel que OH, COOH, PO₃H₂ ou SO₃H, (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
où cation représente Na, K, NR₄ ou/et PR₄ et
où R, indépendamment les uns des autres, de manière identique ou différente, représente aryle comportant jusqu'à 18 atomes de C ou/et (C₁-C₁₈)-alkyle,
et où au moins l'un des substituants de S', S", S"', S"" et S""' est chlore, brome ou iode,
où S', S", S"', S"" ou/et S""', en association les uns avec les autres, forment en option un ou plusieurs anneaux aliphatiques ou/et aromatiques,
ou/et où S', S", S"', S"" ou/et S""' forment en option un pont sur au moins une autre pyridine de la formule générale (III),
ou/et où les radicaux S', S", S"', S"" ou/et S""' présentent en option la signification mentionnée ci-avant et sont substitués par au moins un radical qui présente la signification mentionnée ci-avant pour S', S", S"', S"" ou/et S""', dans de l'eau en présence d'un alcool, d'une base, d'un catalyseur de palladium et en option, d'un ou de plusieurs solvants supplémentaires à une température de 0-200°C.

2. Procédé selon la revendication 1, **caractérisé en ce que** S', S", S"', S"" et S""', indépendamment les uns des autres, de manière identique ou différente, représentent hydrogène, méthyle, éthyle, isopropyle, méthoxy, acétoxy, trifluoroacétoxy, trifluorométhyle, O-phényle, phényle, fluor, OH, nitroso, NO₂, CN, COOH, CHO, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), CONH₂, CO-alkyl-(C₁-C₈), NHCOH, CO-phényle, CHCHCO₂H, PO-phényle₂, PO-alkyl₂-(C₁-C₄), (COO⁻)ₙ(cation)ⁿ⁺, (PO₃²⁻)ₙ(cation)₂ⁿ⁺ (SO₃⁻)ₙ(cation)ⁿ⁺ ou/et (O⁻)ₙ(cation)ⁿ⁺,
où cation représente Na, K, NR₄ ou/et PR₄ et
où R, indépendamment les uns des autres, de manière identique ou différente, représente aryle comportant jusqu'à 18 atomes de C ou/et (C₁- C₁₈)-alkyle.

3. Procédé selon la revendication 1, **caractérisé en ce que**, pour la préparation de 2,2'-bipyridyles symétriques de la formule générale (IV), des 3,3'-bipyridyles symétriques de la formule générale (V) ou des 4,4'-bipyridyles symétriques de la formule générale (VI) 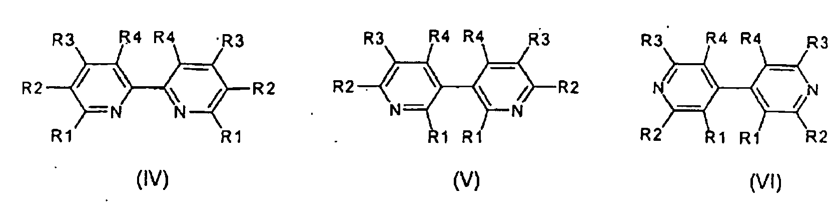 dans lesquelles
R1 à R4, indépendamment les uns des autres, de manière identique ou différente, représentent hydrogène, (C₁-C₁₈)-alkyle, alkoxy-(C₁-C₁₈), acyloxy-(C₁-C₁₈), aryloxy, aryle, hétéroaryle, fluor, hydroxyle, nitro, nitroso, CN, COOH, CHO, PO₃H₂, SO₃H, SO₂R, SOR, NH₂, NH-alkyl-(C₁-C₁₈), N-alkyl₂-(C₁-C₁₈), amine protégée, CF₃, NHCO-alkyl-(C₁-C₄), N-alkyl-(C₁-C₄)-CO-alkyl-(C₁-C₄), COO-alkyl-(C₁-C₁₈), CONH₂, CO-alkyl-(C₁-C₁₈), NHCOH, NHCOO-alkyl-(C₁-C₄), CO-aryle, COO-aryle, CHCH-CO₂-alkyl-(C₁-C₁₈), CHCHCO₂H, PO-phényle₂, PO-alkyl₂-(C₁-C₄), (COO⁻)ₙ(cation)ⁿ⁺, (PO3²⁻)ₙ(cation)₂ⁿ⁺, (SO3⁻)ₙ(cation)ⁿ⁺ ou/et (O⁻)ₙ(cation)ⁿ⁺, où cation représente un métal alcalino-terreux, un métal alcalin, NR₂H₂, NR₃H, NRH₃, NR₄, NH₄, PR₂H₂, PR₃H, PRH₃, PR₄ ou/et PH₄, et aryle, aryloxy et hétéroaryle représentent des radicaux comportant jusqu'à 18 atomes de C,
où R, indépendamment les uns des autres, de manière identique ou différente, représente aryle comportant jusqu'à 18 atomes de C ou/et (C₁-C₁₈)-alkyle,
où R1 à R4, en association les uns avec les autres, forment en option un
ou plusieurs anneaux aliphatiques ou/et aromatiques
et où au moins l'un des substituants de R1 à R4 est un substituant non inerte tel que acyloxy-(C₁-C₈), hydroxyle, COOH, PO₃H₂, SO₃H, SO₂R, NH₂, NH-alkyl-(C₁-C₈), N-alkyl₂-(C₁-C₈), amine non inerte protégée, NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-aryle comportant jusqu'à 18 atomes de C, CHCH-CO₂-alkyl-(C₁-C₈), CHCHCO₂H, (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
ou/et où R1, R2, R3 ou R4 du premier anneau forme en option un pont sur R1, R2, R3 ou R4 du second anneau,
ou/et où les radicaux R1 à R4 présentent en option la signification mentionnée ci-avant et sont substitués par au moins un radical qui présente la signification mentionnée ci-avant pour R1 à R4,
des 2-halopyridines de la formule générale (VII), des 3-halopyridines de la formule générale (VIII) ou des 4-halopyridines de la formule générale (IX), 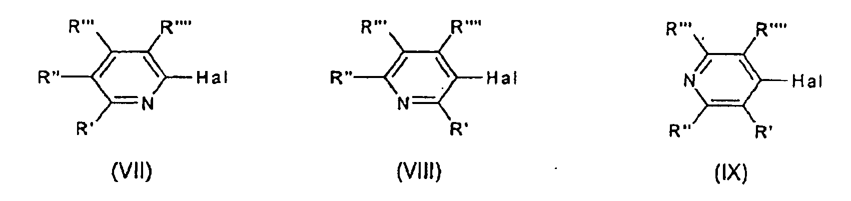 dans lesquelles R', R", R"' et R"" présentent la signification mentionnée ci-avant pour R1 à R4 et où Hal représente chlore, brome ou iode, sont amenés à réagir dans de l'eau en présence d'un alcool, d'une base, d'un catalyseur de palladium et en option, d'un ou de plusieurs solvants supplémentaires à une température de 0-200°C.

4. Procédé selon la revendication 3, **caractérisé en ce que** R1 à R4, indépendamment les uns des autres, de manière identique ou différente, représentent : hydrogène, C₁-C₈-alkyle, alkoxy-(C₁-C₈), acyloxy-(C₁-C₈), aryloxy, aryle, hétéroaryle, fluor, hydroxyle, nitro, nitroso, CN, COOH, CHO, PO₃H₂, SO₃H, SO₂R, SOR, NH₂, NH-alkyl-(C₁-C₈), N-alkyl₂-(C₁-C₈), amine protégée, CF₃, NHCO-alkyl-(C₁-C₄),
N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), CO-aryle, COO-aryle, CHCH-CO₂-alkyl-(C₁-C₈), CHCHCO₂H, PO-phényle₂, PO-alkyl₂-(C₁-C₄), (COO⁻)ₙ(cation)ⁿ⁺, (PO₃²⁻)ₙ(Cation)₂ⁿ⁺, (SO₃⁻)ₙ(cation)ⁿ⁺ ou/et (O⁻)ₙ(cation)ⁿ⁺,
où cation représente Na, Li, K, Ca, Mg, NR₃H, NR₄, NH₄, PR₃H, PR₄ ou/et PH₄ et aryle, aryloxy et hétéroaryle représentent des radicaux comportant jusqu'à 18 atomes de C et où R, indépendamment les uns des autres, de manière identique ou différente, représentent aryle comportant jusqu'à 18 atomes de C ou/et (C₁-C₁₈)-alkyle,
et où au moins l'un des substituants de R1 à R4 est un substituant non inerte, tel que acyloxy-(C₁-C₈), hydroxyle, COOH, PO₃H₂, SO₃H, SO₂R, NH₂, NH-alkyl-(C₁-C₈), N-alkyl₂-(C₁-C₈), amine non inerte protégée, NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-aryle comportant jusqu'à 18 atomes de C, CHCH-CO₂-alkyl-(C₁-C₈), CHCHCO₂H, (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
où cation représente Na, Li, K, Ca, Mg, NR₃H, NR₄, NH₄, PR₃H, PR₄ ou/et PH₄, et où R, indépendamment les uns des autres, de manière identique
ou différente, représente aryle comportant jusqu'à 18 atomes de C ou/et (C₁-C₁₈)-alkyle,
où R1 à R4, en association les uns avec les autres, forment en option un
ou plusieurs anneaux aliphatiques ou/et aromatiques
ou/et où R1, R2, R3 ou R4 du premier anneau forme en option un pont sur R1, R2, R3 ou R4 du second anneau
ou/et où les radicaux R1 à R4 présentent en option la signification mentionnée ci-avant et sont substitués par au moins un radical présentant la signification mentionnée ci-avant pour R1 à R4.

5. Procédé selon la revendication 3, **caractérisé en ce que** R1 à R4, indépendamment les uns des autres, de manière identique ou différente, représentent : hydrogène, méthyle, éthyle, tert-butyle, isopropyle, méthoxy, acétoxy, trifluoroacétoxy, trifluorométhyle, O-phényle, phényle, fluor, OH, nitroso, NO₂, CN, COOH, CHO, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), CO-phényle, COO-phényle, CHCH-CO₂-alkyl-(C₁-C₈), CHCHCO₂H, PO-phényle₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
où cation représente Na, Li, K, NR₃H, NR₄, PR₃H ou/et PR₄ et
où R, indépendamment les uns des autres, de manière identique ou différente, représente aryle comportant jusqu'à 18 atomes de C ou/et (C₁ - C₁₈)-alkyle,
et au moins l'un des substituants de R1 à R4 est un substituant non inerte tel que acétoxy, trifluoroacétoxy, OH, COOH, PO₃H₂, SO₃H, NH₂, NH-alkyl-(C₁-C₈), N-alkyl₂-(C₁-C₈), NHCO-alkyl-(C₁-C₄), N-alkyl-(C₁-C₄)-CO-alkyl-(C₁-C₄), COO-alkyl-(C₁-C₈), CONH₂, CO-alkyl-(C₁-C₈), NHCOH, NHCOO-alkyl-(C₁-C₄), COO-phényle, CHCH-CO₂-alkyl-(C₁-C₈), CHCHCO₂H, (COO⁻)ₙ(cation)ⁿ⁺, (PO₃²⁻)ₙ(cation)₂ⁿ⁺, (SO₃⁻)ₙ(cation)ⁿ⁺ou/et (O-)ₙ(cation)ⁿ⁺,
où cation représente Na, Li, K, NR₃H, NR₄, PR₃H ou/et PR₄ et
où R représente (C₆-C₁₈)-aryle ou/et (C₁-C₁₈)-alkyle, de manière identique ou différente,
où R1 à R4, en association les uns avec les autres, forment en option un ou plusieurs anneaux aliphatiques ou/et aromatiques
ou/et où R1, R2, R3 ou R4 du premier anneau forme en option un pont sur R1, R2, R3 ou R4 du second anneau
ou/et où les radicaux R1 à R4 présentent en option la signification mentionnée ci-avant et sont substitués par au moins un radical qui présente la signification mentionnée ci-avant pour R1 à R4.

6. Procédé selon la revendication 3, **caractérisé en ce que** R1 à R4, indépendamment les uns des autres, de manière identique ou différente, représentent : hydrogène, méthyle, éthyle, isopropyle, méthoxy, acétoxy, trifluoroacétoxy, trifluorométhyle, trichlorométhyle, O-phényle, phényle, fluor, OH, nitroso, NO₂, CN, COOH, CHO, PO₃H₂, SO₃H, NH₂, NH-alkal-(C₁-C₈), N-alkyl₂-(C₁-C₈), NHCO-alkyl-(C₁-C₄), N-alkyl-(C₁-C₄)-CO-alkyl-(C₁-C₄), CONH₂, CO-alkyl-(C₁-C₈), NHCOH, CO-phényle, CHCHCO₂H, PO-phényle₂, PO-alkyl₂-(C₁-C₄), (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
où cation représente Na, K, NR₄ ou/et PR₄ et
où R, indépendamment les uns des autres, de manière identique ou différente, représente aryle comportant jusqu'à 18 atomes de C ou/et (C₁-C₁₈)-alkyle,
et où au moins l'un des substituants de R1 à R4 est un substituant non inerte tel que OH, COOH, PO₃H₂ ou SO₃H, (COO⁻)ₙ(cation)ⁿ⁺, (PO₃²⁻)ₙ(cation)₂ⁿ⁺, (SO₃⁻)ₙ(cation)ⁿ⁺ ou/et (O⁻)ₙ(cation)ⁿ⁺,
où cation représente Na, K, NR₄ ou/et PR₄ et
où R représente aryle comportant jusqu'à 18 atomes de C ou/et (C₁-C₁₈)-alkyle, de manière identique ou différente,
où R1 à R4 en association les uns avec les autres forment en option un ou plusieurs anneaux aliphatiques ou/et aromatiques
ou/et où R1, R2, R3 ou R4 du premier anneau forme en option un pont sur R1, R2, R3 ou R4 du second anneau
ou/et où les radicaux R1 à R4 présentent en option la signification mentionnée ci-avant et sont substitués par au moins un radical présentant la signification mentionnée ci-avant pour R1 à R4.

7. Procédé selon l'une des revendications 1 à 6, **caractérisé en ce que** les sels des halopyridines sont utilisés pour la réaction.

8. Procédé selon l'une des revendications 1 à 7, **caractérisé en ce que** les radicaux R', R", R"' ou/et R"" sont substitués.

9. Procédé selon la revendication 8, **caractérisé en ce que** les radicaux R', R", R"' ou/et R"" sont substitués par les radicaux R1 à R4.

10. Procédé selon l'une des revendications 1 à 8, **caractérisé en ce que**, lors de l'utilisation de deux monohalopyridines différents ou plus, des bipyridyles couplés de façon asymétrique sont également produits en plus des bipyridyles symétriques.

11. Procédé selon l'une des revendications 1 à 10, **caractérisé en ce que**, lors de l'utilisation des halopyridyles, une proportion d'eau dans le milieu de réaction d'au moins 35% est utilisée.

12. Procédé selon l'une des revendications 1 à 11, **caractérisé en ce que** le catalyseur de palladium qui est utilisé est un palladium non supporté ou/et supporté, où un palladium métallique et également un composé de palladium, en option en combinaison l'un avec l'autre, peuvent être utilisés.

13. Procédé selon la revendication 12, **caractérisé en ce que** les supports qui sont utilisés pour le palladium sont du charbon actif, des oxydes métalliques ou/et des sels métalliques.

14. Procédé selon la revendication 12 ou 13, **caractérisé en ce que** le chargement du palladium sur le support est d'au moins 5% en poids.

15. Procédé selon l'une des revendications 12 à 14, **caractérisé en ce que** le catalyseur de palladium, dans chaque cas comme calculé sur la base du métal palladium, est utilisé selon une quantité de 0,0001 à 10 mol% sur la base de halopyridine.

16. Procédé selon l'une des revendications 1 à 15, **caractérisé en ce que** l'alcool utilisé peut seulement subir une réaction aldol avec difficulté ou pas du tout, après oxydation selon l'aldéhyde ou le cétone.

17. Procédé selon l'une des revendications 1 à 16, **caractérisé en ce que** la base utilisée est constituée par des sels de métaux alcalins ou/et de métaux alcalino-terreux.

18. Procédé selon l'une des revendications 1 à 17, **caractérisé en ce que** la réaction est mise en oeuvre à une température de 0 à 200°C et à une pression de 0,1 à 2 MPa.

19. Procédé selon l'une des revendications 1 à 18, **caractérisé en ce que** le produit de réaction comprend de l'acide 2,2'-bipyridyl-4,4'-dicarboxylique.

20. Procédé selon l'une des revendications 1 à 19, **caractérisé en ce que** le produit de réaction est amené à réagir avec un métal de transition pris parmi un sous-groupe 7 ou/et 8 ou/et au moins un métal noble pour obtenir au moins un complexe bipyridyle/métal.

21. Utilisation d'un produit de réaction qui est fabriqué au moyen d'un procédé selon la revendication 20 au niveau de cellules photovoltaïques ou photoélectromécaniques ou pour une électrolyse photo-induite.
